# EUROPEAN PATENT APPLICATION

(11) **EP 3 769 636 A1**
(43) Date of publication of application: **27.01.2021**
(21) Application number: 19188068.1
(22) Date of filing: 24.07.2019
(51) Int. Cl.: A41D 13/11, A61F 5/44, A61F 13/02, A62B 9/00, A62B 18/00

(54) **ARTICLE COMPRISING AN ELONGATED PRESSURE SENSITIVE COMPONENT**

(71) Applicant: 3M Innovative Properties Company, Saint Paul, MN 55133-3427 (US)
(72) Inventor: Urban, Mr. Henning, 331 02 Värnamo (SE); Goenner, Emily S., Shoreview, Minnesota 55126 (US)
(74) Representative: Vollmers, Hans-Gerd

(57) **Abstract**

The invention relates to an article comprising an elongated pressure sensitive component in contact with a user. The pressure sensitive component forms a seal 7 between the user and the article when a minimum seal pressure is applied on the pressure sensitive component. The seal 7 comprises a signal pathway or an inductive loop indicative of a complete seal between user and article at the minimum seal pressure applied along the entire length of the pressure sensitive component.

## Description

The present invention relates to an article comprising an elongated pressure sensitive component in contact with a user, wherein the pressure sensitive component forms a seal between the user and the article.

The protection performance of a personal protective device relies on the filter, impact shielding or sound attenuation efficiency of respirator, eye or hearing protection devices respectively, and on how the devices fit the user (usually a human being). While filter, impact performance and attenuation efficiencies of these protection devices can be controlled solely by material design, the fit of the device to the human being depends additionally on the human factors, such as behaviour and anatomy of the user, including head and body shape, texture and hairiness of the skin, correct wearing of the device etc.

In order to meet the protection requirements of personal protective devices to date, companies rely on protection performances specified by the manufacturer of the protection device, but no regulations or tools exist to determine how well these devices protect the workers in their work environment during working hours. For hearing and respiratory protection devices, there are at least tools to measure sound attenuation within the ear canal behind the hearing protector and aerosol concentrations in the breathing space of a respirator respectively. Only in the case of respiratory protection devices, however, standardized respiratory fit test regulations exist that industries have to follow to protect their workers. Since these respirator fit tests are usually carried out during working hours with clean-shaven "subjects" by an external agency on a yearly basis, these tests tend to be costly and take merely a snapshot of how the respirators fit the user during an entire shift. Hence traditional fit tests are unable to recognize improper use of respiratory protection devices and whether users are shaven during working hours. Traditional respiratory and hearing protector fit tests are therefore unreliable for determining the exposure levels of contaminants or noise levels that a user is exposed to in the working environment over the course of a year. To be able to determine whether the protective equipment truly fits and protects the worker during working hours, a fit measurement is needed to monitor the level of fit of the protection device in real time.

The prior art, such as WO 2015/196255 or US 2015/224275, already disclose systems that have been developed for example for detecting fit of a medical Continuous Positive Airway Pressure (CPAP) device on the patient's facial skin via a multitude of sensors placed on the sealing gasket of the CPAP device. These systems detect contact of the sealing gasket with the patient's skin via the electrical response of the sensors with regard to distance or force between the sealing gasket and the patient's skin. These devices however are only able to detect a seal to the user's skin in locations where the sensors are placed on the gasket. The sections of the gasket between these sensors remain unmonitored with regard to seal of the gasket to the skin.

Other documents disclose this principle of using local sensors to detect fit of a personal protection device or dressing at a location or several numbers of certain locations. Examples of such documents are WO 2015/128173 and US 2016/184538.

In contrast to localised sensor embodiments in gaskets, WO 2007/98762 and WO 2012/28836 disclose systems that monitor leakage of a dressing, which is applied to a surface of an at least partially electrically conductive object, wherein the method comprises the step of detecting changes of the capacitance between a first and second electrode on the electrically conductive object.

WO 2012/28836 discloses a respirator with one or more electrodes of e.g. a conductive elastomer disposed in the surface of a face sealing member opposite to the surface which seals against the user's face. In use, the integrity of the seal formed between the sealing member and the user's face is monitored by monitoring surveying the electric capacitance across the member between the electrodes.

In view of the cited prior art, there is still a need for providing a solution for detecting and/or monitoring the fit of a seal or gasket in its entirety relative to a user of an article, for example the seal of a personal protection device along the entire seal of the protection device with the skin of the user, wherein the detection mechanism is independent of the moisture level or electrical properties of the skin of the user and is able to cope with the different pressure levels around anatomical shapes of the user.

The present invention provides an article comprising an elongated pressure sensitive component in contact with a user, wherein the pressure sensitive component forms a seal between the user and the article when a minimum seal pressure is applied on the pressure sensitive component. The minimum seal pressure is dependent on the anatomical shape variation of the user in the region where the pressure sensitive component is in contact with the user and is determined in a trial with a broad range of subjects. The pressure response of the material is designed to be highly non-linear around the minimum seal pressure, e.g. via the Young's Modulus of the pressure-sensitive component, so that a maximum pressure response at loss of the seal is guaranteed. According to the invention the seal comprises a signal pathway or an inductive loop indicative of a complete seal applied along the entire length of the pressure sensitive component.

The article of the invention may be a personal safety device such as a respirator, hearing protector, eyewear, continuous positive airway pressure (CPAP) face masks, headphones, or hearing aids or helmet, wherein the pressure sensitive component is embedded in the skin-contacting seal, or harness of the personal safety device. The article according to the invention may also be a device out of the health care area such as for example a drug inhaler, wherein the pressure sensitive component is embedded in the nozzle of the drug inhaler and is compressed by a user's mouth when in use. A purpose of the invention is to maintain pressure levels of these above listed components on the skin below a level of discomfort, while assuring a complete seal and assisting in finding more comfortable ways to wear these products.

The elongated pressure sensitive component in contact with the user may be any kind of deformable elongated component that is able to seal a gap that might exist between the article and a user, such as for example a user's skin. The sealing function may occur as soon as a minimum seal pressure is applied on the article and therewith also on the elongated pressure sensitive component. The minimum seal pressure may for example be applied on the elongated pressure sensitive component through all kind of well-known means for fixing an article such as for example a personal protection device to a human body. Such means may include any kind of head bands, harnesses, hoods or temples. For a correct function of the seal it is important to prevent leakage through the seal (complete seal) thereby applying the minimum seal pressure along the entire length of the elongated pressure sensitive component and therewith compressing the seal along the entire length to ensure that no gap occurs along the seal.

According to the invention, the seal which is formed by the elongated pressure sensitive component comprises a signal pathway or an inductive loop that are indicative of the minimum seal pressure applied along the entire length of the pressure sensitive component. The signal pathway may be a pathway for different kind of signals transported by different kind of media, such as for example electrical signals, acoustic signals or optical signals. Depending on the kind of signal used for the invention, different kind of pathways may be selected. For electrical signals, conductive pathways may be selected, for acoustic signals acoustic pathways may be selected and for optical signals optically transparent pathways may be selected. From a construction standpoint, it does not make a big difference if the elongated pressure sensitive component comprises a signal pathway or an inductive loop. The only difference between the two alternatives is that for a signal pathway electrodes or transmitters and detectors are needed and that a inductive loop is be inductively coupled to a resonant circuit in proximity. This will be described in more detail below.

The above described article provides the advantage that it comprises technical features that allow for a real-time assurance of a correct sitting seal (or complete seal) and therefore of protecting the user. Those features may all be integrated into the seal of the article which provides the benefit that an article providing the above-mentioned features does not differ in its geometry a lot from known articles. Advantage of the invention is further that it does not rely on multiplexing or micro-processing of signals from multiple sensors.

According to one embodiment of the invention, the signal of the signal pathway may be based on electric signals such as for example electric currents, electric potential differences, electrical impedances, electrical resistance or electrical capacitance. The signal of the signal pathway may also be based on optical or acoustic signals such as for example transmission, reflection, absorption, scattering and time delays of optical or acoustic waves. The signal of the inductive loop may be based on electrical inductance or inductive coupling of the closed inductive loop to an electric circuit. It is possible to transmit the signal of the pathway and of the inductive loop either remotely to a monitoring digital infrastructure or to an indicator locally on the article itself. Signalling via electric signals, optical and acoustic signals are well known techniques for gathering and transmitting information. They therefore provide a reliable technique for the above-mentioned purpose of the invention.

The following is a list of methods that may be used in the context of the invention in order to get the needed information out of the above-mentioned signals. a) impedance measurement across the two electrodes, b) resonance frequency of inductive coupling, c) time delay & intensity measurements with acoustic receiver, d) intensity measurement with optical receiver, e) operating optical & acoustic transmitter, f) comparing signal to a minimum seal pressure threshold to generate a "loss of seal alert".

According to another embodiment the elongated pressure sensitive component comprises electrodes or terminals for the for measuring electrical currents, electric potential differences, electrical impedance, electrical resistance, electrical capacitance or the like along the signal pathway, or a closed inductive loop coupled to a resonant circuit. The elongated pressure sensitive component may also comprise a transmitter as well as a receiver for sending and receiving optical or acoustic wave signals through the signal pathway. The optical or acoustic wave signals may also be transmitted and received through a transceiver. These components need to be small enough so that they can be integrated into the elongated pressure sensitive component or into the article without limiting its use.

If the pressure sensitive component follows a closed boundary for example on one of the above devices, the two electrodes or terminals may be arranged in close proximity to each other. They may be separated by an insulating part or medium such as for example a gas, fluid or a solid material. With such a configuration the electric signals need to travel through the elongated pressure sensitive component along the entire boundary of the device to get from one electrode to the other.

The electrodes may for example be formed by two consecutive conductive patches that cannot be bridged due to the lack of a conductive patch between them but that are in contact with the signal pathway or conductive loop.

The elongated pressure sensitive component of the article may comprise two opposing structures of aligned conductive patches (opposing patch structures) wherein the two opposing patch structures are separated by an insulating material as long as the minimum seal pressure is not applied onto the pressure sensitive component. The insulating material needs to be selected such that as soon as the minimum pressure is applied along the entire length of the pressure sensitive component, the insulating material separating the two opposing structures disappears or is completely displaced from the space between the two opposing patch structures, such that the two opposing structures can touch each other as soon as the minimum seal pressure is reached. The insulating material may for example be a fluid or a gas. When the minimum seal pressure is reached along the entire length of the pressure sensitive component, the two opposing structures touch each other through the entire length of the pressure sensitive component. The two opposing patch structures may be positioned on two opposing electrically insulating substrates.

The two opposing patch structures may be held apart through spacer elements that are arranged on both sides of the opposing patch structures within the pressure-sensitive component. The spacer elements may provide more stiffness and stability to the seal of the pressure-sensitive component. The spacer elements need to be designed such that they are compressible enough so that they do not interfere with the patches in the two opposing patch structures as they move towards each other and allow each patch to electrically bridge two patches of the opposing patch structure. The spacer elements may be made from any compressible material. The spacer elements may be non-conductive.

The conductive patches in each of the opposing patch structures may be positioned on an insulating substrate or spaced from each other by an insulating compressible material. The conductive patches on the insulating substrate or with the insulating material between them may also form an array of repeating units.

The conductive patches of one of the opposing patch structures are aligned with the gaps between the conductive patches on the opposing insulating substrate or with the insulating material between the conductive patches of the opposing structure so that when the minimum seal pressure is applied along the entire length of the elongated pressure sensitive component each of the conductive patches electrically bridges the opposing insulating material of the opposing patch structures and thereby forms a complete conductive signal pathway between the electrodes at each end of the opposing patch structures or a fully closed inductive loop in the pressure sensitive component. For such a scenario it is important that the extension of the conductive patches is bigger than the extension of the insulating material between the conductive patches.

The electrodes embedded in the conductive patch structure may be formed by two consecutive patches (electrode patches) that are spaced apart from each other exactly like the other patches in the patch structure but missing a conductive patch opposite of the insulating space between them so that the two electrode patches cannot be bridged under compression. It is also possible that the two electrode patches are spaced apart from each other by a distance that is greater than the width of the opposing patch so that the opposing patch cannot bridge the contact between the two electrode patches.

The conductive patches may comprise metal, conductive elastomers, conductive foams, conductive nonwovens, or other conductive compressible or uncompressible materials. The elastomers, foams, nonwovens, or other conductive materials of the conductive patches may comprise conductive filler materials, such as particles of fibers made of metal, graphene, graphite or the like. The elastomer or the conductive patches may comprise silicone or thermoplastic elastomers or viscoelastic gels.

Under the minimum seal pressure applied on the pressure sensitive component each of the conductive patches electrically bridges the opposing insulating material of the opposing patch structure and thereby forms a conductive signal pathway between the electrodes or closes to an inductive loop. As soon as the minimum seal pressure is applied along the entire length of the pressure sensitive component and as soon as all the conductive patches of both opposing structures touch each other it is possible for an electric charge and hence an electrical signal to travel from one electrode to the other electrode along all the conductive patches in the elongated pressure sensitive component or through all the conductive patches within the closed inductive loop in the elongated pressure sensitive component. This electrical signal may be measured at the electrodes in form of an electrical current, a voltage, a change in electrical impedance of the pressure sensitive component, where the electrical impedance is known to be a function of electrical resistance and electrical reactance of the material. This signal may be used as information about the status of the seal. It is an indication that along the entire length of the pressure sensitive component the minimum seal pressure is applied. If the opposing structures are used as an inductive loop, it is also possible to detect a signal in an according resonant circuit. Here again, the signal can only be detected as soon as the signal pathway is closed which means that on the entire length of the pressure sensitive component at least the minimum seal pressure is applied.

The pressure sensitivity and hence the minimum seal pressure threshold are determined by the elastic stiffness or Youngs Modulus of the spacer material, e.g. of the surrounding foam spacer elements or through the amount (pressure) of insulating gas or fluid in the cavity between opposing patches, in which case the gas or fluid take the function of the spacer material to hold the opposing patch structures apart in the uncompressed state and need to be electrically insulating so that the gas or fluid themselves do not create any electrical conduction pathways between the patches in the opposite patch structure.

Instead of opposing conductive patches, it is also possible that the elongated pressure sensitive component comprises a bridging structure that may for example consist of an array of adjacent conductive lamellae that are spaced apart from each other as long as the minimum seal pressure is not applied to the pressure sensitive component. The lamellae may be attached to one or both of opposing non-conductive substrates within the pressure-sensitive component. The lamellae may be designed such as to deform as soon as the minimum seal pressure is applied on the pressure sensitive component. The bridging structures may also comprise any other kind of structures such as for example wall sections, or complex pyramid and wedge structures or the like, which are by design uniquely distributed in the pressure-sensitive component to follow the contour and functional constraints of a gasket in a specific sealing application.

The lamellae may be arranged parallel to each other. They may also be arranged with an angle between them. The lamellae may be or rectangular shape. They may also have more complex shapes, such as for example wedge-like structures or structures with pre-curved surfaces. The lamellae may also be surrounded by a non-conductive fluid or a gas. Air is one example of a gas that could be used. The gas or the fluid needs to have the possibility to be displaced when a pressure gets applied onto the bridging structure.

Spacer elements may be placed next to the lamellae structure within the pressure-sensitive component to provide additional stiffness or stability to the seal of the pressure-sensitive component with the user. The spacer elements need to be compressible so that they allow a deformation of the bridging structure. They may be non-conductive.

When the minimum seal pressure is applied along the entire length of the pressure sensitive component the lamellae in the bridging structure may deform and thereby displacing the surrounding gas or fluid and bridging the space between them resulting either in a complete conductive signal pathway between the electrodes at each end of the bridging structure or in closing to an inductive loop in the pressure sensitive component. In the case where the lamellae are attached to both opposing substrates in the pressure-sensitive component, the bridging functionality of the lamellae may be achieved if the distance between the adjacent lamellae is for example less than half of the combined width of two consecutive lamellae in their deformed state. In the case where the lamellae are attached to only one of the opposite substrates within the pressure-sensitive component, the bridging functionality of adjacent lamellae may be achieved if the distance between them is shorter than their height. The depth of these lamellae-type structures would need to be deep enough to keep the direction of buckling or deforming towards the adjacent lamella.

The electrodes of the lamellae bridging structure may be formed by two consecutive lamellae that are spaced apart from each other and separated through a non-conductive material. It is also possible that they are spaced apart from each other by a distance that is greater than half of the combined width of two consecutive lamellae in their deformed state.

The conductive lamellae may consist of the same materials as listed above for the conductive patches. The lamella terminals may form an electrical connection through at least one of the two planes that the lamellae are attached to.

The elongated pressure sensitive component may comprise a compressible conductive foam structure across the entire length of the elongated pressure sensitive component in the form of a closed inductive loop or with two embedded electrodes at each end of the conductive foam. The two electrodes mentioned above may be embedded at each end of the foam structure.

As soon as the minimum seal pressure is applied along the entire length of the pressure sensitive component the conductive foam generates an electrical signal, wherein the signal may be a change of electrical impedance, due to change in electrical resistance and/or capacitance, between two electrodes embedded in the foam, or a change of inductance in the inductive loop formed by the foam in the elongated pressure sensitive component.

The conductive foam may consist of the same materials mentioned above for the conductive patches and/or the lamellae of the bridging structure. Conductive foam materials may be based on elastomers such as thermoplastics and silicones that have been compounded or coated with electrically conductive materials and are convenient to be implemented in the described applications.

The elongated pressure sensitive component may comprise a conductive non-woven structure across the entire length of the elongated pressure sensitive component in the form of a closed inductive loop or with two embedded electrodes at each end of the conductive non-woven. The conductive non-woven may be compressible. As soon as the minimum seal pressure is applied along the entire length of the pressure sensitive component the conductive non-woven generates an electrical signal, wherein the signal may be an electric current, a voltage or a change of electrical impedance, due to a change in electrical resistance and/or capacitance, between two electrodes embedded in the non-woven, or a change of inductance in the inductive loop formed by the non-woven in the elongated pressure sensitive component.

The fiber of the conductive non-woven may comprise a conductive polymer or a polymer that is loaded with conductive material. The polymer may be a thermoplastic. The polymer may comprise a first polymer that is sheathed with a second polymer which is loaded with conductive material. Both first and second polymers may be a thermoplastic such as a polypropylene, polyethylene or any other suitable polymer or a combination thereof. The conductive material within the fiber or on the surface of the fiber may comprise the form of a particle, of a fiber or a tube or of a film. It may consist of metal, carbon, graphite, polymer, inorganic oxides, other electrically conductive materials or a combination thereof.

The non-woven may have a density of 25 gsm and consists of fibers with polypropylene core, polyethylene sheath, wherein the fibers have been loaded with graphite particles and carbon fibers.

The elongated pressure sensitive component may also comprise two opposing, complementary structures that consist of a material that is optically transparent to the wave from an optical transmitter or acoustically transparent to the wave from an acoustic transmitter, where the optical or acoustic transmitter and an optical or acoustic receiver are each embedded at the same end or opposite ends of one of the opposing complementary structures. The surface of the complementary structures may have any shapes as long as the two shapes of the two opposing structures in the pressure-sensitive component fit exactly to each other when brought into contact with each other, which means that at contact there is no gap or space between the opposing complementary structures anymore. One example of such opposing complementary structures may be zigzag structures. But all other structures such as for example more complex structures or structures with more curvatures are possible too as long as they are complementary as described above.

The two opposing structures may be held apart through spacer elements that are arranged on both sides of the opposing structures within the pressure-sensitive component. The spacer elements may provide more stiffness or stability to the seal of the pressure-sensitive component with the user. The spacer elements need to be designed such that they are compressible and that when compressed do not interfere with the two opposing structures that need to be able to move towards each other and touch each other. The spacer elements may be made of any compressible material. It may be non-conductive. If in contact with the opposing complementary structures, the spacer material would need to be of different refractive index or mass density or bulk modulus than the material of the opposing complimentary structure.

The two opposing, complementary structures may be filled with a fluid or gas, wherein the fluid or gas between the complementary structures may have a refractive index that is different from the refractive index of the material of the complementary structures at the wavelength of an optical or acoustic transmitter or it may have a mass density or bulk modulus that is different from the mass density and bulk modulus of the material of the complementary structures. The fluid or gas may get displaced as soon as a pressure is applied on to the two opposing, complementary structures.

When the minimum seal pressure is applied along the entire length of the elongated pressure sensitive component the opposing, complementary structures engage with each other and thereby displace all the gas or fluid producing a waveguide for optical or acoustic waves. A signal is thereby generated, wherein the signal is produced by the optical or acoustic receiver receiving the optical or acoustic wave through the waveguide from the optical or acoustic transmitter respectively, wherein the optical or acoustic wave may be reflected at one end of the waveguide if the optical or acoustic transmitter share the same end of the waveguide with the optical or acoustic receiver respectively is displaced and the two structures are in direct contact with each other or engage with each other such that an optical or acoustic wave can travel along the elongated pressure sensitive component in which the two opposing complementary structures produce a waveguide for optical or acoustic waves. An optical or acoustic receiver embedded at the opposite or same end of the waveguide as the optical or acoustic transmitter is able to detect the optical or acoustic wave travelling through the waveguide. If the optical or acoustic receiver shares the same end of the waveguide with the optical or acoustic transmitter then the receiver is able to detect optical or acoustic waves reflecting at the end of the waveguide respectively. As long as the minimum amount of applied pressure is not reached over the entire length of the elongated pressure sensitive component the optical or acoustic waveguide will not be fully assembled between the optical or acoustic transmitter and receiver, causing the optical or acoustic wave to scatter at the interfaces between complimentary structure and surrounding gas or fluid and preventing it from coupling into the waveguide, or causing the acoustic wave to traverse fluid or gas in the unassembled waveguide sections of the pressure sensitive component creating a measurable time delay for the acoustic waves to reach the receiver.

The waveguide that is assembled from the opposing complimentary structures in the pressure-sensitive component at minimum seal pressure may be a classic waveguide where the waves travel along the waveguide via total internal reflection. The assembled waveguide in the pressure-sensitive component may also form a metamaterial which have an optical or acoustic band structure that confines optical or acoustic waves to the waveguide instead of following the classic rule of total internal reflection. Metamaterials are often based on repeating material patterns, such as repeating pores in a material matrix or repeating patterns of different materials in a material matrix and may be based on photonic or phononic crystals. The repeating patterns are usually on a length scale smaller than the wavelength of the optical or acoustic wave. Such a metamaterial waveguide would be assembled at the minimum seal pressure along the entire pressure-sensitive component just as described above. If the minimum seal pressure threshold is not reached anywhere along the pressure-sensitive component, then the metamaterial waveguide cannot assemble in that region and is unable to guide the optical or acoustic wave to the receiver.

The materials of the opposing complementary structures may consist of metals, ceramics, inorganic materials, or polymers, or a combination thereof. The polymers may be rigid, elastomeric, or viscoelastic, such as for example thermoplastics, silicones, gels etc., and may be compounded with filler material to adjust its mass density. Filler materials may be metals, ceramics, inorganic oxides or nitrides, or other materials with uniform mass distribution and mass density different from the polymer resin. The material of the opposing complimentary structures may also be a porous material as described below, where one of the above-mentioned materials contain pores that are filled with the gas or fluid, which may deform under compression and displace the gas or fluid.

The pressure sensitive component may also comprise a porous structure, such as a foam or mesh structure, made of a material with low enough optical absorption (optically transparent) or low enough acoustic attenuation (acoustically transparent) for optical or acoustic waves that are transmitted from an optical or acoustic transmitter to an optical or acoustic receiver over the entire length of the pressure sensitive component respectively, where the optical or acoustic transmitter and an optical or acoustic receiver are each embedded at the same end or opposite ends of the compressible porous structure. Possible materials for such a porous structure may be for example optically clear polymers, such as thermoplastic elastomers or silicones, or resins filled with filler materials with mass density higher than the polymer resin, such as metals, ceramics inorganic oxides or nitrides, or others.

The cells in the porous structure may be filled with a fluid or gas, wherein the fluid or gas has a refractive index that is different from the refractive index of the material of the porous structure at the wavelength of the optical or acoustic transmitter or the fluid or gas has a mass density and bulk modulus that is different from the mass density and bulk modulus of the porous structure.

Under the minimum seal pressure applied along the entire length of the elongated pressure sensitive component the porous structure may get compressed thereby displacing the fluid or gas and building a waveguide for optical and acoustic waves. If the minimum seal pressure is applied a signal is generated, wherein the signal is produced by optical or acoustic waves traveling with lower scattering losses through the waveguide from optical or acoustic transmitter to optical or acoustic receiver compared to the uncompressed state of the porous structure or by acoustic waves traveling at a different speed through the waveguide from the acoustic transmitter to the acoustic receiver compared to the uncompressed state of the porous structure, wherein the optical or acoustic wave may be reflected at one end of the waveguide if the optical or acoustic transmitter share the same end of the waveguide with the optical or acoustic receiver respectively. For this to happen the porous structure may contain pores that are interconnected so that fluid or gas can flow through the pores. For optical and acoustic waves, the compressed porous structure becomes a transparent waveguide allowing light or acoustic waves to travel with minimal scattering losses from transmitter to receiver in the pressure sensitive component, resulting in a light intensity or acoustic intensity dependent pressure response at the optical or acoustic receiver respectively. The compression of the porous structure results also in a higher mass density and more uniform mass distribution in the compressed porous structure compared to the uncompressed state allowing acoustic waves to travel at a different speed of sound and with lower losses from the transmitter to the receiver through the elongated pressure sensitive component as in an uncompressed state, resulting in an additional measurable time and intensity dependent pressure response at the acoustic receiver.

The porous structure may be a foam produced by established moulding and foaming techniques or it may be a mesh structure produced by additive manufacturing processes, such as 3D printing, stereolithography, selective laser sintering, or others.

The waveguide that is assembled from the compressed porous structure in the pressure-sensitive component at minimum seal pressure may be a classic waveguide where the waves travel along the waveguide via total internal reflection. The assembled waveguide in the pressure-sensitive component may also form a metamaterial which have an optical or acoustic band structure that confines optical or acoustic waves to the waveguide instead of following the classic rule of total internal reflection. Metamaterials are often based on repeating material patterns, such as repeating pores in a material matrix or repeating patterns of different materials in a material matrix and may be based on photonic or phononic crystals. The repeating patterns are usually on a length scale smaller than the wavelength of the optical or acoustic wave. Such a metamaterial waveguide would be assembled at the minimum seal pressure along the entire pressure-sensitive component just as described above, where the repeating patterns or repeating pores remain in the compressed state of the porous structure. If the minimum seal pressure threshold is not reached anywhere along the pressure-sensitive component, then the metamaterial waveguide cannot assemble in that region and is unable to guide the optical or acoustic wave to the receiver.

The invention also relates to a method of using an electronic circuit to measure electric current, voltage, or electrical impedance shift across the electrode terminals in the elongated pressure sensitive component, where the electrical impedance is a function of the electrical resistance and reactance of the elongated pressure sensitive component and where a threshold current, threshold voltage, or threshold impedance are indicative of a complete seal of the elongated pressure sensitive component with the user's skin.

The invention also relates to a method of using the inductance or resonance frequency shift of the inductive loop in the elongated pressure sensitive component, which is measured by an electronic circuit which is coupled inductively to the inductive loop in the elongated pressure sensitive component, where the inductance or threshold resonance frequency of the inductive loop are indicative of a complete seal of the elongated pressure sensitive component with the user's skin.

The invention also relates to a method of using an electronic circuit to measure light intensity with a light detector at the end of an optically transparent opposing complementary structure or an optically transparent porous structure in the elongated pressure sensitive component, whereby a threshold light intensity is indicative of an optical waveguide being fully built by these optical structures and of a complete seal of the elongated pressure sensitive component with the user's skin.

And finally, the invention also relates to a method of using an electric circuit to measure intensity shifts and time delays of acoustic pulses with an acoustic receiver at the end of an acoustically transparent opposing complementary structure or an acoustically transparent porous structure in the elongated pressure sensitive component , whereby a threshold intensity or threshold time delay of acoustic signals may be indicative of an acoustic waveguide being fully built by these acoustic structures and of a complete seal of the elongated pressure sensitive component with the user's skin.

The invention will now be described in more detail with reference to the following Figures exemplifying particular embodiments of the invention:
- Fig. 1: a schematical view of a seal or gasket placed onto a skin of a user;
- Fig. 1A: cross-sectional view along the longitudinal axis of an elongated pressure sensitive component with two opposing structures of conductive patches;
- Fig. 1B: cross-sectional view along the transvers axis of the elongated pressure sensitive component of figure 1a;
- Fig. 2A: cross-sectional view along the longitudinal axis of the elongated pressure sensitive component of figure 1a with a force F1 applied to it;
- Fig. 2B: cross-sectional view along the longitudinal axis of the elongated pressure sensitive component of figure 1a with a force F2 applied to it;
- Fig. 3A: cross-sectional view along the longitudinal axis of an elongated pressure sensitive component with a structure of parallel conductive lamellae;
- Fig. 3B: cross-sectional view along the transvers axis of the elongated pressure sensitive component of figure 3a;
- Fig. 4A: cross-sectional view along the longitudinal axis of the elongated pressure sensitive component of figure 3a with a force F1 applied to it;
- Fig. 4B: cross-sectional view along the longitudinal axis of the elongated pressure sensitive component of figure 3a with a force F2 applied to it;
- Fig. 5A: cross-sectional view along the longitudinal axis of an elongated pressure sensitive component with a conductive foam structure;
- Fig. 5B: cross-sectional view along the transvers axis of the elongated pressure sensitive component of figure 5a;
- Fig. 6A: cross-sectional view along the longitudinal axis of the elongated pressure sensitive component of figure 5a with a force F1 applied to it;
- Fig. 6B: cross-sectional view along the longitudinal axis of the elongated pressure sensitive component of figure 5a with a force F2 applied to it;
- Fig. 7A: cross-sectional view along the longitudinal axis of an elongated pressure sensitive component with conductive non-woven;
- Fig. 7B: cross-sectional view along the transvers axis of the elongated pressure sensitive component of figure 7a;
- Fig. 8A: cross-sectional view along the longitudinal axis of the elongated pressure sensitive component of figure 7a with a force F1 applied to it;
- Fig. 8B: cross-sectional view along the longitudinal axis of the elongated pressure sensitive component of figure 7a with a force F2 applied to it;
- Fig. 9A: cross-sectional view along the longitudinal axis of an elongated pressure sensitive component with two opposing complementary zigzag structures made of acoustically transparent material with a force F2 applied to it;
- Fig. 9B: cross-sectional view along the transvers axis of the elongated pressure sensitive component of figure 9a;
- Fig. 10: cross-sectional view along the longitudinal axis of the elongated pressure sensitive component of figure 9a with a force F1 applied to it;
- Fig. 11A: cross-sectional view along the longitudinal axis of an elongated pressure sensitive component with acoustically transparent foam material with a force F2 applied to it;
- Fig. 11 B: cross-sectional view along the transvers axis of the elongated pressure sensitive component of figure 11 a;
- Fig. 12: cross-sectional view along the longitudinal axis of the elongated pressure sensitive component of figure 11a with a force F1 applied to it;
- Fig. 13A: cross-sectional view along the longitudinal axis of an elongated pressure sensitive component with two opposing complementary zigzag structures made of optically transparent material;
- Fig. 13B: cross-sectional view along the transvers axis of the elongated pressure sensitive component of figure 13a;
- Fig. 14A: cross-sectional view along the longitudinal axis of the elongated pressure sensitive component of figure 13a with a force F1 applied to it;
- Fig. 14B: cross-sectional view along the longitudinal axis of the elongated pressure sensitive component of figure 13a with a force F2 applied to it;
- Fig. 15A: cross-sectional view along the longitudinal axis of an elongated pressure sensitive component with two parallel waveguides made of optically transparent material and a divider;
- Fig. 15B: cross-sectional view along the transvers axis of the elongated pressure sensitive component of figure 14a showing the two parallel waveguides and the mechanical divider;
- Fig. 16A: cross-sectional view along the longitudinal axis of the elongated pressure sensitive component of figure 15a with a force F1 applied to it,
- Fig. 16B: cross-sectional view along the longitudinal axis of the elongated pressure sensitive component of figure 15a with a force F2 applied to it, and
- Fig. 17: a diagram showing typical pressure response curves of different materials over compressive stress.

Herein below various embodiments of the present invention are described and shown in the drawings wherein like elements are provided with the same reference numbers.

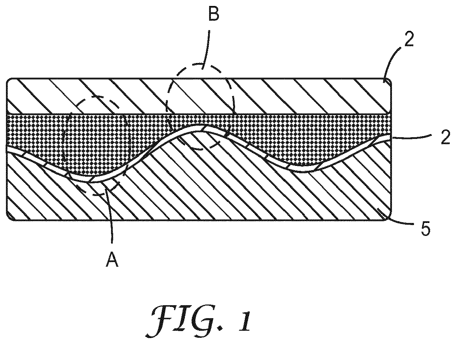

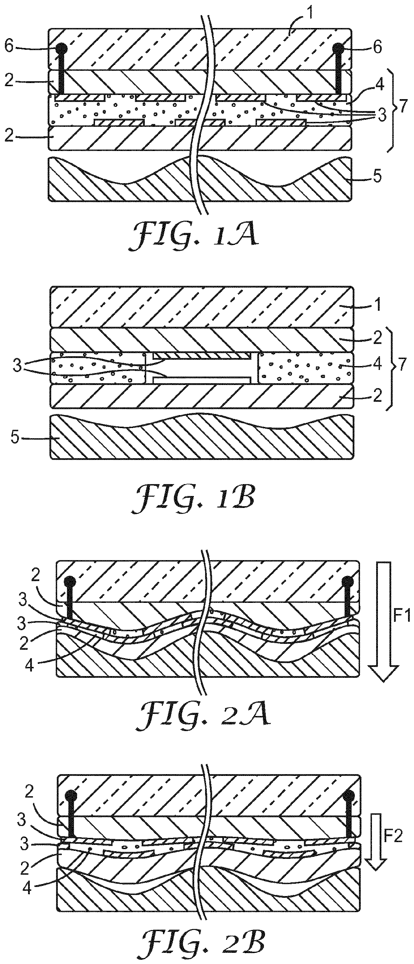

Figure 1 a schematical view of a seal or gasket 2 placed onto a skin 5 of a user. The gasket 2 provides a hollow space in between two layers. In areas A, where the two layers 2 of the gasket are spaced apart from each other a minimum seal pressure zone is established. In areas B, where the two layers 2 of the gasket are positioned close to each other a maxim seal pressure is established. The minimal and maximal seal pressure of the pressure-sensitive seal component depend not only on the force applied across the area of the seal, but also locally on the anatomical shape in contact with the gasket. Using the pressure-sensitive component to determine fit of the seal the minimum pressure is essential to determine a complete seal of the gasket. Hence the minimum pressure is used for the seal pressure threshold.

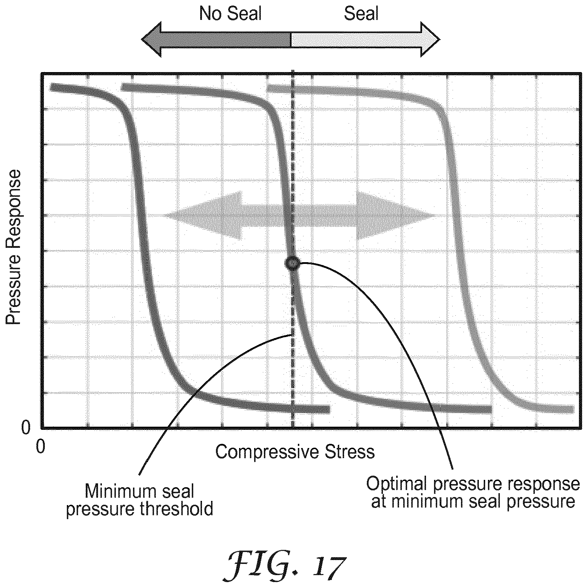

Figure 1A is a cross-sectional view along the longitudinal axis of an elongated pressure sensitive component with two opposing structures of conductive patches. The elongated pressure sensitive component may for example be a seal for sealing the gap between a personal protective device, such as for example a respirator mask, a hearing protector or eyewear, and a person's skin or any other kind of seal (see Figures 17A through 18C). Figure 1A shows part of the protection device 1 to which the pressure sensitive component is attached to. The protection device 1 is followed by a first gasket layer 2. Next to the gasket or seal layer 2 a first layer of subsequent conductive patches 3 is arranged wherein the conductive patches 3 are flat and spaced apart from each other. With a certain distance a second layer of subsequent conductive patches 3 is arranged wherein the conductive patches 3 of the second layer are also spaced apart from each other. The space between the patches 3 as well as the space between the first layer of patches 3 and the second layer of patches 3 is filled with a non-conductive material, such as a gas for example air. The two layers of conductive patches 3 may be held apart from each other through compressible spacer elements 4 that can be seen in Figure 1B and that are positioned on each side of it. The two layers of conductive patches 3 are arranged such relative to each other that each conductive patch 3 is arranged opposite of a space between two conductive patches 3. Also, the extension of the space between two patches 3 is smaller than the extension of the patches 3. The second layer of conductive patches 3 is again followed by a second gasket layer 2 which is in contact with skin 5 of a person. The first gasket layer 2, the first layer of conductive patches 3, the spacer 4, the second layer of conductive patches 3 and the second gasket layer 2 may form a seal 7 of a personal protection device 1. The seal may also be a closed seal on the sides of the spacer elements 4, such that it would show with a squared cross section in Figure 1B. On both ends of the elongated pressure sensitive component a terminal 6 is positioned. Each of the terminals 6 is in contact with one conductive patch 3. The terminals themselves are separated from each other.

Figure 1B, which is a cross-sectional view along the transvers axis of the elongated pressure sensitive component of Figure 1A, shows that the compressible spacers 4, that are arranged on both sides of the conductive patches 3, keep the conductive patches apart as long as no pressure is applied onto the elongated pressure sensitive component. The compressible spacers 4 may be also the sidewalls of the gasket.

As soon as a pressure in form of a force F is applied onto the elongated pressure sensitive material, the spacer elements 4 get compressed and the two layers of conductive patches 3 get closer to each other. Upon a certain force F1 (see Figure 2A) all conductive patches 3 get in contact with each other thereby building a conductive path between the two terminals 6 through the elongated pressure sensitive component, which can trigger a signal directly at the terminals 6 or by forming an inductive loop (not shown) that is coupled to a remote resonant circuit. This conductive path may be used directly as an electric circuit via the terminals 6 and is able to trigger a visual or audible indication of a complete seal without the need of multiplexing or micro-processing the signal. A time event may be communicated to an internal or wireless data logging device.

If the pressure sensitive material shown in the Figures 1A to 2B is used as a seal 7 for example for a personal protection device the force F may get applied onto the seal upon skin contact and may exert a compressive stress to the gasket 2 and the spacer 4 within the seal 7. Due to the alignment of spacer 4 and electrode patches 3 the stresses in the spacer 4 need to be large enough to compress the spacer 4 by nearly 100% in order for the conductive patches 3 to be able to form an electrical contact. The compressive and flexural modulus and thickness of the spacer 4 and seal layer 2 allow control of the compressive strain as result of the compressive stress. A partial gasket 2 skin 5 contact due to human factors such as body shape, movement, or hair on the skin surface would - as for example shown in Figure 2B - result in a reduction of the compressive stress and consequently the strain in the spacer 4 so that not all conductive patches 3 are able to form an electrical contact with the according opposite conductive patches 3. As a result, the electrode patches 3 cannot form a complete electrical conduction path and the circuit across the terminals remain open (see Figure 2B).

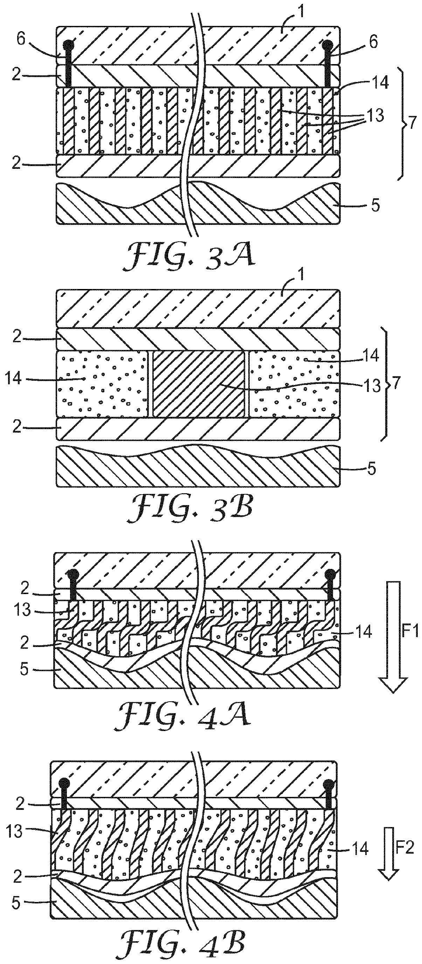

Figure 3A is a cross-sectional view along the longitudinal axis of an elongated pressure sensitive component with a structure of essentially parallel conductive lamellae 13. The elongated pressure sensitive component may for example be a seal for sealing the gap between a personal protective device, such as for example a respirator mask, a hearing protector or eyewear, and a person's skin. Figure 3A shows part of the protection device 1 to which the pressure sensitive component is attached to. The protection device 1 is followed by a first gasket layer 2. Next to the gasket or seal layer 2 the structure of conductive lamellae 13 is arranged wherein the lamellae 13 are arranged parallel and spaced apart from each other such that the lamellae - when no pressure is applied on the elongated pressure sensitive component - do not touch each other. The lamellae 13 may consist of a mechanically compliable and electrically conductive polymer compound as explained in the general part of the description. The space between the lamellae 13 is filled with a non-conductive gas or fluid, such as air for example. The lamellae 13 extend between the first gasket layer 2 and a second parallel gasket layer 2. They may be fixed with their upper most end to the first gasket layer 2 and with their lower end to the second gasket layer 2. In the uncompressed state the two layers of gasket 2 may be held apart by the lamellae themselves or through compressible spacer elements 14 that can be better seen in Figure 3B. The second gasket layer 2 is in contact with skin 5 of a person. The first gasket layer 2, the structure of lamellae 13, the spacer elements 14 and the second gasket layer 2 may form a seal 7 of a personal protection device 1. On both ends of the elongated pressure sensitive component a terminal 6 is positioned. Each of the terminals 6 is in contact with one lamella 13 of the lamellae structure. Also, in this embodiment the seal 7 may be closed at the sides of the spacer elements 14.

Figure 3B, which is a cross-sectional view along the transvers axis of the elongated pressure sensitive component of Figure 3A, shows that the spacers 14 that are arranged on both sides of the lamellae 13 to keep the two gasket layers 2 apart as long as no force is applied to the elongated pressure sensitive component. The spacers 14 may also act as sidewalls of the gasket.

As soon as a pressure in form of a force F is applied onto the elongated pressure sensitive material, the spacers 14 get compressed and the lamellae 13 get bended. The higher the applied force F is the more the lamellae 13 are bent. Upon a certain force F1 all lamellae 13 are bent so much that they get in contact with each other thereby building a conductive path through the elongated pressure sensitive component between the two terminals 6, which can trigger a signal directly at the terminals 6 or by forming an inductive loop (not shown) that is coupled to a remote resonant circuit (see Figure 4A). This conductive path may be used directly as an electric circuit via the terminals 6 and is able to trigger a visual or audible indication of a complete seal without the need of multiplexing or micro-processing the signal. A time event may be communicated to an internal or wireless data logging device.

If the pressure sensitive material shown in the Figures 3A to 4B is used as a seal 7 for a personal protection device the force F may get applied onto the seal upon skin contact and may exert a compressive stress to the gasket 2 and the spacer elements 14 within the seal 7. Due to the alignment of spacers 14 and the lamellae structure 13 the stresses in the spacers 14 needs to be large enough to compress the spacers 14 in order for the lamellae 13 to be able to contact each other and to form an electrical path. The compressive and flexural modulus and thickness of the spacers 14 and gasket materials 2 allow control of the compressive strain as result of the compressive stress. A partial gasket 2 to skin 5 contact due to human factors such as body shape, movement, or hair on the skin surface would result in a reduction of the compressive stress and consequently the strain in the spacers 14 so that not all lamellae 13 are able to form an electrical contact with the according adjacent lamellae 13. As a result, the lamellae structure 13 cannot form a complete electrical conduction path and the circuit across the terminals remains open (see Figure 4B).

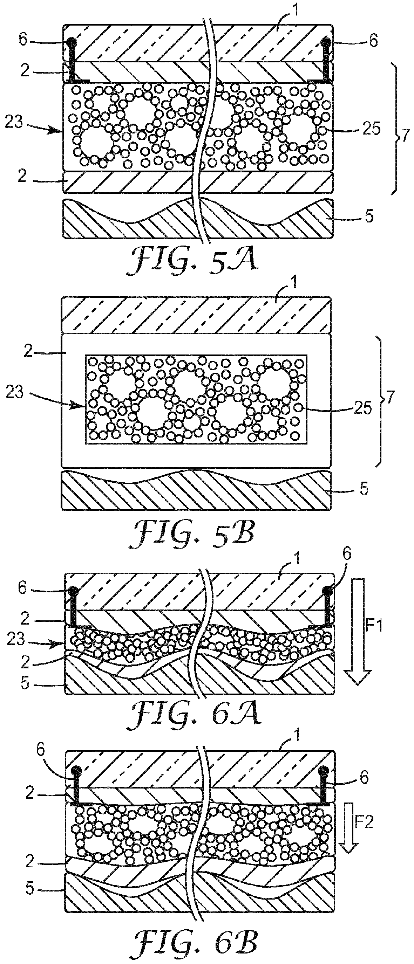

Figure 5A is a cross-sectional view along the longitudinal axis of an elongated pressure sensitive component with a conductive foam structure 23. The conductive foam structure 23 may consist of a mechanically compliable polymer that comprises an electrically conductive filler material 25, such as micro-particles, nanotubes or nanofibers. The elongated pressure sensitive component may for example be a seal for sealing the gap between a personal protective device, such as for example a respirator mask, a hearing protector or eyewear, and a person's skin. Figure 5A shows part of the protection device 1 to which the pressure sensitive component is attached to. The protection device 1 is followed by a first gasket layer 2. Next to the gasket layer 2 the conductive foam structure 23 is arranged wherein the foam structure 23 extends parallel to the first gasket layer 2. The cells within the foam structure 23 are filled with a non-conductive gas or fluid, such as for example air. The conductive foam structure 23 extends between the first gasket layer 2 and a second parallel gasket layer 2. The two layers of gasket 2 may be held apart from each other through the gasket sidewalls in figure 5b, the compressible, conductive foam structure 23 and/or through additional spacers as in the other embodiments (not shown in the drawings). The second gasket layer 2 is in contact with skin 5 of a person. The first gasket layer 2, the conductive foam structure 23 and the second gasket layer 2 may form a seal 7 of a personal protection device 1. On both ends of the elongated pressure sensitive component a terminal 6 is positioned. The two terminals 6 are in contact with the conductive foam structure 23. The gasket 2 may also comprise a hollow space filled with the conductive foam structure 23 as can be seen in Figure 5B.

Figure 5B, which is a cross-sectional view along the transvers axis of the elongated pressure sensitive component of Figure 5A, shows that the conductive foam structure 23 is surrounded by gasket sidewalls 2 also on the left and right. The gasket sidewalls, the conductive foam structure 23 and/or additional spacer material as in the other embodiments (not shown in this drawing) keeps all the gasket layers 2 separated from each other in the uncompressed state.

As soon as a pressure in form of a force F is applied onto the elongated pressure sensitive material, the conductive foam structure 23 gets compressed thereby reducing the size of its cells and contacting the electrically conductive filler material 25 within the foam structure 23 and therewith reducing the electrical resistance of the foam structure 23 which closes the circuit between the two terminals 6. The higher the applied force F is the more the foam structure 23 gets compressed. Upon a certain force F1 a conductive path through the elongated pressure sensitive component is build, which can trigger a signal directly at the terminals 6 or by forming an inductive loop (not shown) that is coupled to a remote resonant circuit (see Figure 6A). This conductive path may be used directly as an electric circuit via the terminals 6 and is able to trigger a visual or audible indication of a complete seal without the need of multiplex or micro-processing the signal. A time event may be communicated to an internal or wireless data logging device.

If the pressure sensitive material shown in the Figures 5A to 6B is used as a seal 7 for a personal protection device the force F may get applied onto the seal upon skin contact and may exert a compressive stress to the gasket 2 and the conductive foam structure 23 within the seal 7. The compressive and flexural modulus and thickness of the foam structure 23 and gasket materials 2 allow control of the compressive strain as result of the compressive stress. A partial gasket 2 to skin 5 contact due to human factors such as body shape, movement, or hair on the skin surface would result in a reduction of the compressive stress and consequently the strain in conductive foam structure 23 so that less filler material is able to form an electric contact. This increases the electrical resistance in the foam 23 again, which opens the circuit between the terminals (see Figure 6B).

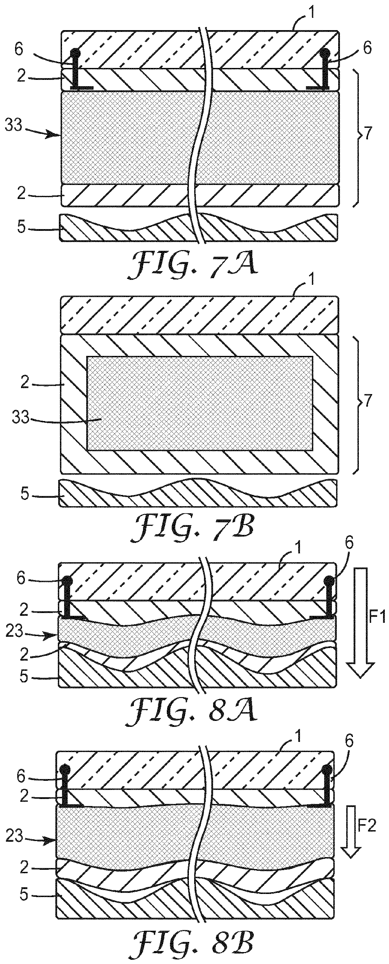

The embodiment shown in Figures 7A to 8B differs from the embodiment shown in the Figures 5A to 6B in that the gasket 2 is not filled with a conductive foam 23 but with an electrically conductive non-woven 33 that has been coated with an electrically conductive material. The electrically conductive non-woven 33 forms an electrical conduction path along the gasket 2 as soon as the gasket is in complete contact with the skin 5 of a person. Just as in the other embodiments in Figures 1A to 6B this conduction path is created due to the electrical impedance change of the compressed nonwoven and may be measured directly via a voltage and current transient by an electric circuit at two terminals 6 or indirectly if the conductive path forms an inductive loop that is coupled to a resonant circuit. The measured signals can be used to trigger a visual or audible fit indication if the electrical impedance exceeds the threshold impedance at the minimum seal pressure determined in a fit test.

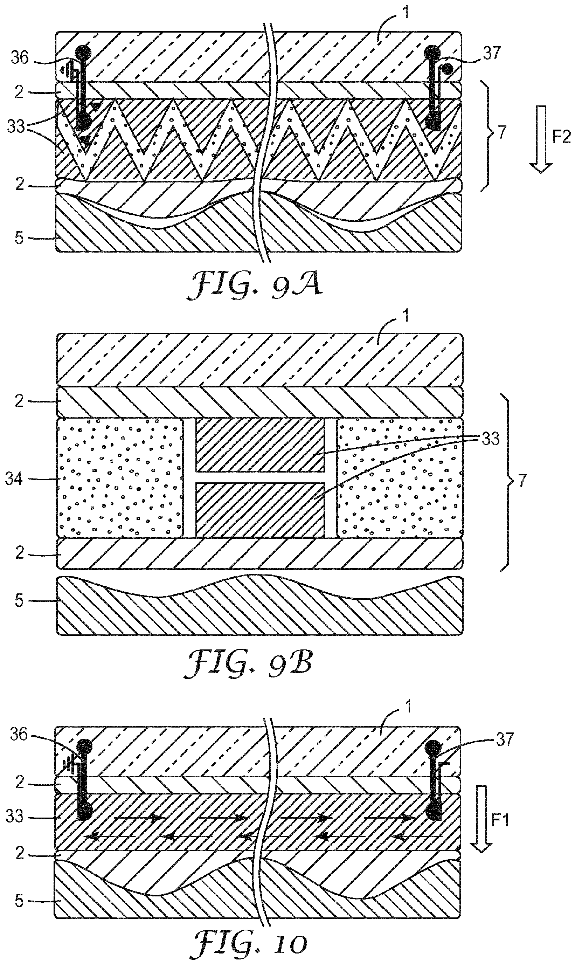

Figure 9A is a cross-sectional view along the longitudinal axis of an elongated pressure sensitive component with two opposing complementary zigzag structures of acoustically transparent material. The elongated pressure sensitive component may for example be a seal for sealing the gap between a personal protective device, such as for example a respirator mask, a hearing protector or eyewear, and a person's skin. Figure 9A shows part of the protection device 1 to which the pressure sensitive component is attached to. The protection device 1 is followed by a first gasket layer 2. Next to the gasket layer 2 a first zigzag structure of acoustically transparent material 33 is arranged. A second corresponding zigzag structure of acoustically transparent material 33 is arranged parallel to the first structure 33 wherein the two structures also are arranged parallel and spaced apart from each other such that they - when no pressure is applied on the elongated pressure sensitive component - do not touch each other or do not engage with each other. The space between the zigzag structures 33 is filled with a fluid or gas such as for example air. The second zigzag structure 33 of acoustically transparent material is followed by a second gasket layer 2, followed by skin 5. The two layers of gasket 2 may be held apart from each other through compressible spacers 34 that can be seen in Figure 9B and/or gasket sidewalls (not shown in this figure). The second gasket layer 2 is in contact with skin 5 of a person. The first gasket layer 2, the two zigzag structures 33, the spacers 34 and the second gasket layer 2 may form a seal 7 of a personal protection device 1. On both ends of the elongated pressure sensitive component a transmitter 36 and a receiver 37 are positioned that are embedded in the first zigzag structure 33.

Figure 9B, which is a cross-sectional view along the transvers axis of the elongated pressure sensitive component of Figure 9A, shows that the spacer elements 34 that are arranged on both sides of the zigzag structures 33 to keep the two gasket layers 2 with the zigzag structures 33 apart from each other as long as no force is applied to the elongated pressure sensitive component. The spacers can also have the function of gasket sidewalls.

As soon as a pressure is applied onto the elongated pressure sensitive material by a force F, the spacers 34 get compressed thereby bringing the two zigzag structures 33 closer to each other and finally into engagement. The higher the applied force F is the closer the zigzag structures get until they are in final engagement with each other. Final engagement means that the two zigzag structures 33 fully engage with each other such that their entire surfaces touch each other, and no gas or fluid is being entrapped between them. In final engagement the two zigzag structures 33 build a waveguide for acoustic waves.

Upon a certain force F1 the two zigzag structures 33 fully engage with each other thereby building the above-mentioned waveguide through the elongated pressure sensitive component (see Figure 10). The acoustic path in the waveguide may be used for guiding acoustic waves from a transmitter 36 to a receiver 37 embedded in the gasket 2. The receiver's response can trigger a visual or audible fit indication without the need of multiplexing or micro-processing the signal. A time event may be communicated to an internal or wireless data logging device.

If the pressure sensitive material shown in the Figures 9A to 10 is used as a seal 7 for a personal protection device the force F may get applied onto the seal upon skin contact and may exert a compressive stress to the gasket 2 and the spacer elements 34 within the seal 7. Due to the alignment of spacers 34 and the zigzag structure 33 the stresses in the spacers 34 need to be large enough to compress the spacers 34 in order for the zigzag structure 33 to be able to engage with each other to form an acoustic waveguide. The compressive and flexural modulus and thickness of the spacers 34 and gasket materials 2 allow control of the compressive strain as a result of the compressive stress. A partial gasket 2 to skin 5 contact due to human factors such as body shape, movement, or hair on the skin surface would result in a reduction of the compressive stress and consequently the strain in the spacers 34 so that there is no engagement over the entire length of the two zigzag structures 33. As a result, the zigzag structures 33 cannot form a complete acoustic waveguide and no response can be generated by the acoustic receiver 37 (see Figure 9A).

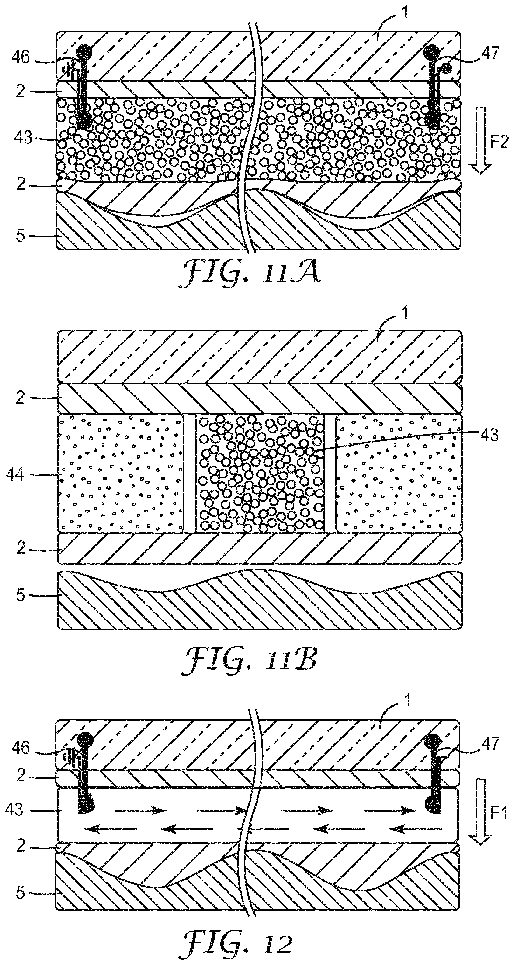

The embodiment shown in Figures 11A to 12 differs from the embodiment shown in the Figures 9A to 10 in that the construction comprises a porous structure in form of a foam 43 that forms an optical or acoustic waveguide upon complete skin contact of the gasket 2 between the first and second gasket layer 2 instead of the two opposing zigzag structures 33. The foam or porous structure may consist of an optically or acoustically transparent polymer, such as a silicone, thermoplastic resin, or other. The voids in the porous structure are filled with a gas or fluid, such as for example air. The embodiment also comprises spacer elements 44 and/or two gasket sidewalls (not shown in this figure) arranged on both sides of the foam or porous structure 43.

The system also includes an optical or acoustic transmitter 46 as well as an optical or acoustic receiver 47. Without any pressure applied to the elongated pressure sensitive material, the foam or porous structure 43 is in an expanded state, in which it is unable to guide an optical or acoustic wave from the transmitter 46 due to scattering of the optical wave at the gas or fluid filled voids or due to insufficient bulk modulus and mass density in the foam or porous structure for the acoustic wave so that no signal is obtained by the receiver 47. As soon as a minimum seal pressure is applied on the elongated pressure sensitive material, an optical or acoustic waveguide is formed by the displaced gas or fluid in the compressed foam or porous structure 43 which may guide optical waves so that the receiver 47 receives signals through the optical waveguide from the transmitter 46. As soon as the receiver 47 receives the signal it may trigger a visual or audible fit indication without the need of multiplexing or micro-processing the signal.

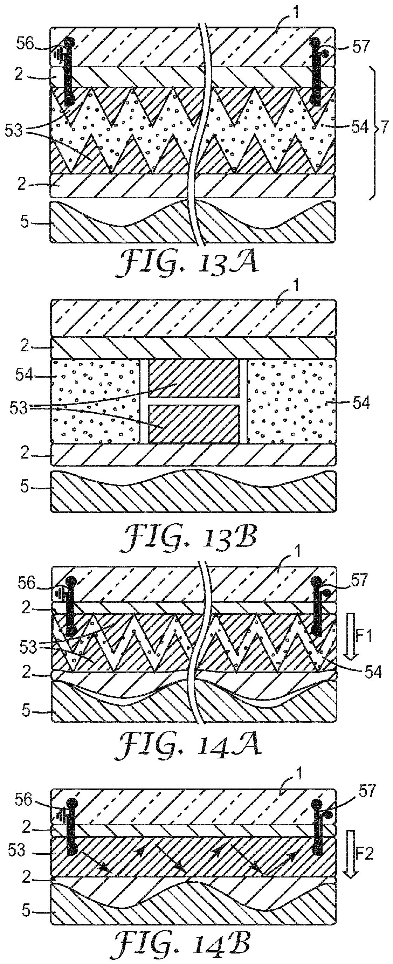

The embodiment shown in Figures 13A to 14B resembles the embodiment shown in Figures 9A to 10. The only difference is that in Figures 13A to 14B optical waves are guided from a transmitter to a receiver instead of acoustic waves. The material used in the embodiment of Figures 13A to 14B are optically transparent materials instead of acoustically transparent materials.

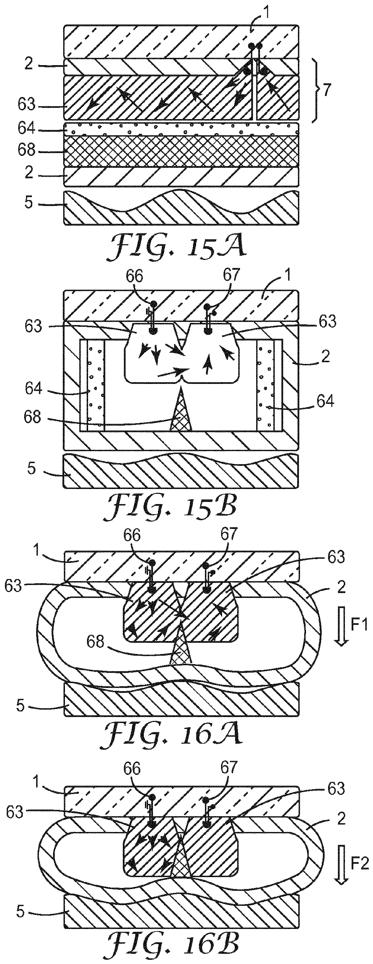

In Figures 15A to 15B another embodiment using optical waves is described. The embodiment again comprises an elongated pressure sensitive component that may be used as seal for a gap between a personal protection equipment and skin of a person. The gasket comprises a first gasket layer 2 attached to the protection device 1 as well as a second gasket layer 2 next to the skin 5 of a person. Within the gasket 2 two parallel optically transparent waveguides 63 are arranged and extend parallel to the extension of the pressure sensitive component. Within the first optically transparent waveguide 63 an optical transmitter 66 is embedded and within the second optically transparent waveguide 63 an optical receiver 67 is embedded. The two parallel waveguides touch each other along their entire length. The gasket 2 further comprise a separator 68 with for example a triangular cross-section as in Figure 15B, which is positioned in the gasket opposite of the two waveguides 63. As soon as a force is applied onto the elongated pressure sensitive component, the gasket 2 gets compressed, thereby moving the separator 68 towards the two parallel waveguides 63 and separating them such that they do not touch each other anymore. And finally, the gasket 2 may comprise two spacers 64, each spacer 64 being arranged on one side of the two waveguides 63.

An optical wave, that is generated and send out by the transmitter 63 may move through the first waveguide 63 and as long as the two waveguides touch each other also through the second waveguide 63, where it may be detected through the receiver 67. As soon as the separator 68 separates the two waveguides 63, the receiver 67 may not detect any optical wave from the transmitter 66 anymore which may be an indication of an applied pressure that has forced the separator between the two waveguides 63. As in all the other embodiments the material and dimensions of the components (gasket 2, spacer 64 etc.) of the system need to be selected such that the receiver 67 gets a signal only if the gasket is not completely sealing the skin towards the personal protection device.

Figure 17 a diagram showing typical pressure response curves of different materials over compressive stress. The pressure response of the pressure-sensitive component is designed to be highly non-linear with regard to the compressive stress from the seal pressure in the pressure-sensitive component. The Young's Modulus of the pressure-sensitive component can be used to match the pressure response of the pressure-sensitive component with the minimum seal pressure threshold of a group of subjects. The midpoint of the pressure response curve is considered to be the optimal match with the minimum seal pressure threshold.

## Claims

1. An article comprising an elongated pressure sensitive component in contact with a user,
wherein the pressure sensitive component forms a seal (7) between the user and the article when a minimum seal pressure is applied on the pressure sensitive component and
wherein the seal (7) comprises a signal pathway or an inductive loop indicative of a complete seal along the entire length of the pressure sensitive component.

2. The article according to claim 1, wherein the signal of the signal pathway is based on electrical signals or on optical or acoustic waves, and wherein the signal of the inductive loop may be based on electrical inductance or inductive coupling of the closed inductive loop to an electric circuit.

3. The article according to claim 1 or 2, wherein the pressure sensitive component comprises a transmitter (36, 46, 56, 66) as well as a receiver (37, 47, 57, 67), or electrodes (6) or terminals.

4. The article according to any of the preceding claims, wherein the elongated pressure sensitive component comprises two opposing structures of aligned conductive patches (3) (opposing patch structures) wherein the two opposing patch structures are separated by an insulating material and as long as the minimum seal pressure is not applied onto the pressure sensitive component.

5. The article according to the claim 4, wherein under the minimum seal pressure applied on the pressure sensitive component each of the conductive patches (3) electrically bridges the opposing insulating material of the opposing patch structure and thereby forms a conductive signal pathway between the electrodes or closes to an inductive loop.

6. The article according to any of the claims 1 to 3, wherein the elongated pressure sensitive component comprises a bridging structure that consists of an array of adjacent conductive lamellae (13) that are spaced apart from each other as long as the minimum seal pressure is not applied to the pressure sensitive component and that are designed such as to deform as soon as the minimum seal pressure is applied on the pressure sensitive component.

7. The article according to claim 6, wherein under the minimum seal pressure applied along the entire length of the pressure sensitive component the lamellae (13) of the bridging structure deform and thereby bridge the space between the lamellae resulting either in a complete conductive signal pathway between the electrodes (6) at each end of the bridging structure or in closing to an inductive loop in the pressure sensitive component.

8. The article according to any of the claims 1 to 3, wherein the elongated pressure sensitive component comprises a compressible conductive foam (23) across the entire length of the elongated pressure sensitive component in the form of a closed inductive loop or with two embedded electrodes (6) at each end of the conductive foam.

9. The article according to claim 8, wherein under the minimum seal pressure applied along the entire length of the pressure sensitive component the conductive foam (23) generates an electrical signal, wherein the signal may be based on an electric current, a voltage, or a change of electrical impedance, due to change in electrical resistance and/or capacitance, between two electrodes (6) embedded in the foam (23), or a change of inductance in the inductive loop formed by the foam in the elongated pressure sensitive component.

10. The article according to any of the claims 1 to 3, wherein the elongated pressure sensitive component comprises a conductive non-woven (33) across the entire length of the elongated pressure sensitive component in the form of a closed inductive loop or with two embedded electrodes (6) at each end of the conductive non-woven.

11. The article according to claim 10, wherein under the minimum seal pressure applied along the entire length of the pressure sensitive component the conductive non-woven (33) generates an electrical signal in, wherein the signal is be based on an electric current, a voltage, or a change of electrical impedance, due to a change in electrical resistance and/or capacitance, between two electrodes (6) embedded in the non-woven or a change of inductance in the inductive loop formed by the non-woven in the elongated pressure sensitive component.

12. The article according to any of the claims 1 to 3, wherein the elongated pressure sensitive component comprises two opposing, complementary structures (33) that consist of a material that is optically transparent to the wave from an optical transmitter (36) or acoustically transparent to the wave from an acoustic transmitter (56), where the optical or acoustic transmitter and an optical or acoustic receiver are each embedded at the same end or opposite ends of one of the opposing, complimentary structures.

13. The article according to claim 12, wherein the space between the two opposing, complementary structures (33) is filled with a fluid or a gas wherein the fluid or gas between the complementary structures (33) has a refractive index that is different from the refractive index of the material of the complementary structures (33) at the wavelength of an optical or acoustic transmitter (36) or wherein the fluid or gas between the complementary structures (33) has a mass density and bulk modulus that is different from the mass density and bulk modulus of the material of the complementary structures (33) and/or wherein under the minimum seal pressure applied along the entire length of the pressure sensitive component the opposing, complementary structures (33) engage with each other, thereby displacing the fluid or gas, producing a waveguide for optical or acoustic waves.

14. The article according to any of the claims 1 to 3, wherein the elongated pressure sensitive component comprises a porous structure (43), made of a material with low enough optical absorption or low enough acoustic attenuation for optical or acoustic waves that are transmitted from an optical or acoustic transmitter to an optical or acoustic receiver over the entire length of the pressure sensitive component and/or wherein the pores in the porous structure (43) are filled with a fluid or a gas, wherein the fluid or gas has a refractive index that is different from the refractive index of the material of the porous structure (43) at the wavelength of an optical or acoustic transmitter (46), or wherein the fluid or gas has a mass density or bulk modulus that is different from the mass density or bulk modulus of the material of the porous structure.

15. The article according to claim 14, wherein under the minimum seal pressure applied along the entire length of the pressure sensitive component the porous structure (43) gets compressed thereby displacing the fluid or gas and producing a waveguide for optical or acoustic waves
